(19) Europäisches Patentamt
European Patent Office
Office européen des brevets

(11) Publication number: **0 480 537 A1**

# (12) EUROPEAN PATENT APPLICATION

(21) Application number: **91202652.3**

(22) Date of filing: **11.10.91**

(51) Int. Cl.[5]: **B01J 23/66**, B01J 23/68, C07D 301/10

(30) Priority: **12.10.90 US 596242**

(43) Date of publication of application: **15.04.92 Bulletin 92/16**

(84) Designated Contracting States: **AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Thorsteinson, Erlind Magnus**
**1112 Highland Road**
**Charleston, W. Virginia 25302(US)**
Inventor: **Minahan, David Michael**
**5416 Tiffany Drive**
**Cross Lanes, W. Virginia 25313(US)**
Inventor: **Liu, Albert Cheng-Yu**
**34 Crestmont Drive**
**Charleston, W. Virginia 25311(US)**

(74) Representative: **Smulders, Theodorus A.H.J., Ir. et al**
**Vereenigde Octrooibureaux, Nieuwe Parklaan 97**
**NL-2587 BN Den Haag(NL)**

(54) **Stable alkylene oxide catalysts.**

(57) Catalysts for the production of alkylene oxide by the epoxidation of alkene with oxygen in the presence of an efficiency enhancing gaseous member of a redox-half reaction pair comprise a silver impregnated support containing an efficiency enhancing amount of at least one efficiency-enhancing salt of a member of a redox-half reaction pair and a sufficient amount of stability-enhancing component to provide enhanced catalytic stability as compared to a similar catalyst which does not contain such stability-enhancing component.

EP 0 480 537 A1

## Field of the Invention

This invention relates to silver-containing, supported catalysts for the epoxidation of alkene, especially ethylene, to the corresponding alkylene oxide, e.g., ethylene oxide, which contain a stability enhancing component comprising at least one of iron, nickel, copper, ruthenium, rhodium, osmium, iridium, at least one of the rare earths, rhenium, antimony, gold, zinc, thallium, lead, tin and cadmium.

## Background to the Invention

Ethylene oxide is commercially produced by the epoxidation of ethylene over silver-containing catalyst at elevated temperature. Considerable research efforts have been devoted to providing catalysts that increase the efficiency, or selectivity, of the process to ethylene oxide.

The manufacture of ethylene oxide by the reaction of oxygen or oxygen-containing gases with ethylene in the presence of a silver catalyst is an old and developed art. For example, U. S. Patent No. 2,040,782, patented May 12, 1936, describes the manufacture of ethylene oxide by the reaction of oxygen with ethylene in the presence of silver catalysts which contain a class of metal-containing promoters. In Reissue U. S. Patent 20,370, dated May 18, 1937, Leforte discloses that the formation of olefin oxides may be effected by causing olefins to combine directly with molecular oxygen in the presence of a silver catalyst. From that point on, the prior art has focused its efforts on improving the catalyst's efficiency in producing ethylene oxide.

In characterizing this invention, the terms "conversion", "selectivity", and "yield" are employed as defined in U. S. Patent No. 3,420,784, patented January 7, 1969, at column 3, lines 24-35 inclusive. This definition of "selectivity" is consistent with that disclosed in U. S. Patent No. 2,766,261 at column 6, lines 5-22, and U. S. Patent No. 3,144,916, lines 58-61. The definitions of "yield" and "conversion" have more varied meaning in the art and are not to be employed as defined, for example, in the aforementioned U. S. Patent No. 2,766,261. The terms "efficiency" and "selectivity", as used throughout the specification and claims are intended to be synonymous.

Researchers have sought catalysts providing higher selectivities and greater conversions to alkylene oxides. At one time researchers believed that the mechanism for alkene epoxidation necessitated conversion of a portion of the alkene to carbon dioxide, i.e., an efficiency plateau existed regardless of catalyst improvements. This plateau was believed to be about 86% for ethylene epoxidation. Thereafter, efficiencies higher than the predicted plateau were observed.

United States Patent No. 4,007,135 discloses processes for the epoxidation of alkene to alkylene oxide. The patent reports high selectivities to alkylene oxides under certain operating conditions. The phenomenon of high selectivity was pursued by researchers. See United States Patent Nos. 4,168,247 and 4,226,782 which describe alkali metal-containing silver catalysts providing selectivities to ethylene oxide of greater than 90 percent. United States Patent No. 4,094,889 notes that catalysts providing the very high selectivities lose selectivity with time, e.g., selectivity declines of 0.2 to 5 percent or more per month.

An advance in the very high selectivity catalysts is described in British Patent Application 2,014,133 which describes an olefin epoxidation process in which olefin and oxygen are contacted in the presence of a silver-containing catalyst and a chlorine-containing reaction modifier, and the selectivity of the catalyst is improved or at least partly maintained or restored by contacting the catalyst with a gas comprising $N_2O_4$ and/or NO or a nitrogen-containing compound together with an oxidizing agent which is preferably oxygen. The catalysts exemplified contained alkali metal-containing compound which is a nitrate salt or likely converted to a nitrate salt under the conditions of the catalyst preparation. The epoxidation system disclosed is thus one in which the silver catalyst contains an efficiency-enhancing salt of a redox half-reaction pair and the gas phase contains an efficiency enhancing gaseous member of a redox half-reaction pair. Even with the addition of the gaseous nitrogen oxide component, the catalysts still exhibited undesirable performance declines with use. For instance, United States Patent No. 4,822,900 reports that nitropropane additives improve the selectivity of the catalyst or reduce the rate of loss of selectivity of the catalyst.

While improved efficiencies of conversion to ethylene oxide are desirable, the typical concomitant increase in temperature (i.e., loss of activity) can be troublesome for a commercially-viable catalyst. Commercial ethylene oxide plants are typically operated to provide a desired balance of productivity and efficiency. Less active catalysts are thus operated at higher temperatures to achieve desired productivity. However, the upper temperature range of the catalyst is limited. Consequently, catalysts that have high initial temperatures for a given conversion rate may have shorter useful lives. Not only is catalyst a major expense to the ethylene oxide plant owner, but also, the plant must be shut down for substantial periods of

time to discharge the old catalyst and charge new catalyst to the typical tubular, fixed bed ethylene oxide reactors. Hence, without a useful lifetime, e.g., two years or more, the benefit of any enhanced efficiency is quickly lost in catalyst replacement costs and plant shut-down time. Thus, the activity stability and/or efficiency stability of a catalyst are important concerns in achieving a commercially viable ethylene oxide catalyst.

Methods are sought to enhance the activity and, in particular, the activity stability and/or efficiency stability of silver-containing, supported ethylene oxide catalysts which have been promoted to enhance efficiency, which while providing desirable efficiencies, are typically less active and must be operated at higher temperatures to be useful in commercial production facilities. These high temperatures can unduly shorten the catalyst life such that the catalysts are unattractive for commercial facilities. Catalysts with enhanced activity stability and/or efficiency stability would be very advantageous.

By way of background as to promoters used in alkene epoxidation catalysts most often used in conventional types of catalysts which do not achieve the desired very high efficiencies, the following discussion is presented.

U.S. Patent 4,007,135 broadly discloses (in column 2, lines 25-30) silver catalysts for alkylene oxide production containing silver "together with a promoting amount of at least one promoter selected from lithium, potassium, sodium, rubidium, cesium, copper, gold, magnesium, zinc, cadmium, strontium, calcium, niobium, tantalum, molybdenum, tungsten, chromium, vanadium and barium...".

U.S. Patent Nos. 3,844,981 and 3,962,285 disclose catalysts and processes for epoxidizing olefins in the presence of a multimetallic component. The catalyst in the 3,962,285 patent is said to comprise a minor amount of one or more of palladium, ruthenium, rhenium, iron and platinum with a major amount of silver. The 3,844,981 patent discloses the preparation of the catalyst from a decomposable salt of group 7b, 1b or the iron group of group 8 of the Periodic Table of the Elements. Preferably, the salt is selected from the group of gold, copper, rhenium, manganese and iron salts. While the patentee contemplates that these metals are in the metallic state, oxidation during epoxidation conditions may occur with one or more of these metals, e.g., rhenium, to form oxyanions containing the metal.

United States Patent No. 2,040,782 discloses silver-containing catalysts for the manufacture of alkylene oxides which catalysts:

"may be considerably enhanced by the admixture with the catalytic material of small quantities of other materials capable of acting as promoters. Suitable promoters, which may be used singly or in combination, include the metals such as copper, gold, iron, manganese, nickel, cobalt, cerium, thorium and zinc." (Page 2, column 1, lines 3 to 9)

United States Patent No. 2,605,239 discloses the use of beryllium oxide as a promoter. Other promoter metals such as copper, aluminum, manganese, cobalt, iron, magnesium, gold, thorium, nickel, cesium and zinc are suggested. These promoter metals are to be incorporated into the catalyst by mechanical mixture or coprecipitation.

United States Patent No. 2,615,900 states:

"The activity of the silver may be enhanced by inclusion in the silver catalyst of promoters such as iron, nickel, copper, gold, platinum, manganese, cobalt, cerium, thorium, zinc, and the oxides, hydroxides,and carbonates of alkali metal and alkaline earth metals." (Column 3, lines 33 to 38)

Japanese patent application Kokai 78/39404 discloses a gas phase process for the epoxidation of three and four carbon atom olefins in the presence of a silver-cadmium-silicon catalyst. The patent applicant states that other components can be incorporated in the catalyst such as elements of Groups I, II, III and VIII such as cesium, copper, gold, magnesium, calcium, beryllium, barium, zinc, aluminum, lanthanum, cerium, zirconium, thorium, iron, cobalt, nickel and platinum.

United States Patent No. 3,758,418 discloses catalysts prepared by a coating technique. Among the catalysts suggested in the patent are those used for the manufacture of ethylene oxide. The metals that can be deposited are said to include the catalytically active metals found in Group IIIb to Va of the Periodic Table.

Japanese patent application Kokai 89/01224047 reported by Chemical Abstracts, Vol. 112 (10):83303f reports a cobalt, iron or nickel catalyst containing silver as a co-catalyst for the decomposition of nitrogen oxides to molecular nitrogen without the addition of ammonia.

U.S. Patents 3,836,481; 3,899,445; and 4,257,967, assigned to Toray Industries, Inc., disclose silver catalyst compositions which include at least one rare earth metal and which are selective in the oxidation of ethylene to ethylene oxide. Such patents do not suggest the use of rare earth metals in the catalyst systems of the present invention.

Bhasin et al U.S. Patent 4,916,243 discloses a supported silver catalyst containing cesium and at least one other alkali metal to increase the efficiency of ethylene oxide manufacture. Bhasin et al U.S. Patent

4,419,276 and Warner et al U.S. Patent 4,455,392 disclose supported silver catalysts for ethylene oxide manufacture. These patents disclose catalyst carriers which include alpha-alumina with the balance being a mixture of silicon dioxide, various alkali oxides, alkaline earth oxides, iron oxide, and other metal and non-metal oxides. No particular catalytic role for iron oxide is disclosed in these patents. Often, such carriers have very minor amounts of iron oxide, e.g., on the order of 5 ppm by weight, which are insufficient to have any substantial effect on the final catalyst.

## Summary of the Invention

By this invention silver-containing, supported alkylene oxide catalysts suitable for use in systems for the epoxidation of alkene to alkylene oxide in which the catalysts comprise at least one efficiency-enhancing salt of a redox-half reaction pair in conjunction with at least one gaseous efficiency-enhancing member of a redox-half reaction pair are provided that have enhanced activity and/or efficiency stability. The catalysts contain a sufficient amount of at least one stability enhancing component selected from the group of iron, nickel, copper, ruthenium, rhodium, osmium, iridium, at least one of the rare earth metals (lanthanide series), rhenium, antimony, gold, zinc, thallium, lead, tin, and cadmium. Such stability enhancing component is present in an amount sufficient to increase the activity stability and/or the efficiency stability of the catalyst as compared to a similar catalyst which does not contain the stability enhancing component under otherwise identical conditions. Often, the stability enhancing component is present in an amount of at least about 10 or 20, e.g., about 25 to 1000, preferably about 50 to 500, ppm (weight) calculated as the weight of the metal of the stability enhancing component based on the total weight of the catalyst. The amount of stability enhancing component which provides the enhanced stability generally varies depending on the specific stability enhancing component employed and on the nature and amounts of other components in the catalyst composition.

The increase in catalyst stability of the present invention is particularly advantageous since it is provided in already high efficiency catalysts, i.e., catalysts which Comprise at least one efficiency-enhancing salt of a redox-half reaction pair which are preferably intended for use in conjunction with at least one gaseous efficiency-enhancing member of a redox-half reaction pair. The combination of components of the present invention which provides both enhanced efficiency and enhanced stability in a single catalyst composition results in very beneficial catalyst and alkene epoxidation process.

The stability of a catalyst can be with respect to at least one of efficiency aging rate and activity aging rate. In a more stable catalyst, the efficiency aging rate and/or activity aging rate is less than that in a less stable catalyst. An especially beneficial attribute of the catalysts of this invention is an enhanced efficiency stability during the epoxidation process. By enhanced efficiency stability, the selectivity of the catalyst to the production of alkylene oxide does not decrease as rapidly over time of operation as would a similar catalyst but which does not contain the stability enhancing component at identical operating conditions.

As used herein, the term "compound" refers to the combination of a particular element with one or more different elements by surface and/or chemical bonding, such as ionic and/or covalent and/or coordinate bonding. The term "ionic" or "ion" refers to an electrically charged chemical moiety; "cationic" or "cation" being positive and "anionic" or "anion" being negative. The term "oxyanionic" or "oxyanion" refers to a negatively charged moiety containing at least one oxygen atom in combination with another element. An oxyanion is thus an oxygen-containing anion. It is understood that ions do not exist in vacuo, but are found in combination with charge-balancing counter ions.

The catalyst contains at least one efficiency-enhancing salt of a redox-half reaction pair promoter in an amount sufficient to enhance the efficiency of the catalyst as compared to a similar catalyst which does not contain the promoter. Preferably, the catalyst comprises alkali metal nitrate, especially potassium and/or rubidium nitrate, especially in amounts greater than about 400 or 500 parts per million (ppm) by weight based on the weight of the catalyst. In this aspect of the invention, a nitrogen and oxygen-containing compound, e.g., nitrogen oxide, nitrogen dioxide, etc., may be introduced into the reaction zone containing the catalyst as a copromoter to enhance at least one of activity, efficiency and stability of the catalyst performance.

An aspect of this invention relates to the use of the aforementioned catalysts in epoxidizing alkene to alkylene oxide, especially ethylene to ethylene oxide.

## Detailed Discussion

Alkylene oxides made using the catalysts of this invention are characterized by the structural formula

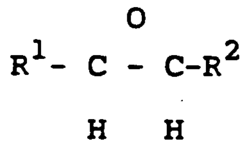

wherein $R^1$ and $R^2$ are lower alkyl, e.g., methyl or ethyl or, preferably, hydrogen. Most preferably the alkylene oxide is ethylene oxide. The alkylene oxides are made from the corresponding alkene, i.e., $R^1HC = CHR^2$. For purposes of ease of understanding, the following discussion will be made with reference to ethylene oxide and ethylene.

The catalysts of this invention are characterized by combining a sufficient amount of at least one stability enhancing component to enhance stability of the catalyst as compared to a similar catalyst which does not contain the stability enhancing component. Although the catalysts can be used under widely varying process conditions, for purposes of determining whether sufficient stability enhancing component has been incorporated into the catalyst, a standard set of process conditions can be used.

The STANDARD ETHYLENE OXIDE PROCESS

CONDITIONS (ABBR. "CONDITIONS") for characterizing the catalysts of this invention involve the use of a standard backmixed autoclave with full gas recycle including carbon dioxide. The CONDITIONS may be operated with some variation in ethylene, oxygen and gas phase inhibitor feed. The case illustrated involves oxygen process conditions, which simulates in the backmixed reactor the typical conditions in commercial oxygen-type ethylene oxide processes where molecular oxygen, as such, is employed. When, as is preferred, the catalyst is intended to be used in conjunction with the corresponding efficiency-enhancing gaseous member of a redox-half reaction pair, the CONDITIONS provide for the presence of such gaseous member. The CONDITIONS employ 2.0 mole % ethylene oxide in the outlet gas of the reactor under the following standard inlet conditions:

| Component | Oxygen process Conditions, |
|---|---|
| Oxygen | 8.0 |
| Ethylene | 30 |
| Ethane | 0.5 |
| Carbon Dioxide | 6.5 |
| Nitrogen | Balance of Gas |
| Parts per million ethyl chloride (or one-half such amount when ethylene dichloride is used) | Optimum for Efficiency |
| Parts per million gaseous member of redox half reaction pair (when required for catalyst) | Optimum for Efficiency |

The CONDITIONS employ the well known backmixed bottom-agitated "Magnedrive" autoclaves described in Figure 2 of the paper by J. M. Berty entitled "Reactor for vapor Phase-Catalytic Studies", in Chemical Engineering Progress, Vol. 70, No. 5, pages 78-84, 1974.

The pressure is maintained constant at 275 psig and the total outlet flow is maintained at 22.6 SCFH. SCFH refers to cubic feet per hour at standard temperature and pressure, namely, 0 ° C and one atmosphere. The outlet ethylene oxide concentration is maintained at 2.0% by adjusting the reaction temperature. Thus temperature ( ° C) and catalyst efficiency are obtained as the responses describing the catalyst performance.

The catalyst test procedure used in the CONDITIONS involves the following steps:

1. 80 cc of catalyst are charged to the backmixed autoclave. The volume of catalyst is measured in a 1 inch I.D. graduated cylinder after tapping the cylinder several times to thoroughly pack the catalyst. The volume of catalyst is alternatively calculated from the packing density of the carrier and the amount of silver and additives. The weight of the catalyst is noted.
2. The backmixed autoclave is heated to about reaction temperature in a nitrogen flow of 20 SCFH with the fan operating at 1500 rpm. The nitrogen flow is then discontinued and the above-described feed stream is introduced into the reactor. The total gas outlet flow is adjusted to 22.6 SCFH. The temperature is set at 220° C.
3. The temperature is raised over the next three days to 255° C. The selectivity and the activity of the catalyst to ethylene oxide are thus obtained.

The standard deviation of a single test result reporting catalyst efficiency in accordance with the procedure described above is about 0.7% efficiency units. The standard deviation of a single test result reporting catalyst activity in accordance with the procedure described above is about 0.03 mole% ethylene oxide. The standard deviation, of course, will depend upon the quality of the equipment and precision of the techniques used in conducting the tests, and thus will vary. The test results reported herein are believed to be within the standard deviation set forth above. The running of a multiplicity of tests will reduce the standard deviation by the square root of the number of tests.

The activity stability and efficiency stability of a catalyst is conveniently determined under the CONDITIONS. The rate of decrease in activity and efficiency with time is indicative of the activity stability and efficiency stability of the catalyst. Usually, the study is conducted for about 50 days with a delta ethylene oxide concentration across the catalyst of about 2 mole percent. The time to provide an indication of stability may be 20 or 30 days at ethylene oxide production rates of about 2 mole percent.

The amount of stability enhancing component is often sufficient to provide an efficiency increase under CONDITIONS of at least about 0.1 or 0.2, preferably at least about 0.5, and more preferably at least about 1, efficiency percentage point after 50 days of operation as compared to a substantially identical catalyst but not containing the stability enhancing component. Additionally, or alternatively, the activity stability of the catalyst may be enhanced by the stability enhancing component. Often, after 50 days of operation under Standard Ethylene Oxide Process Conditions, the activity of a catalyst containing the stability enhancing component is at least about 5 or 10, preferably at least about 20, percent higher than that of an otherwise identical catalyst but not containing the stability enhancing component. The effect of the stability enhancing component appears to be unique to epoxidation systems in which the catalyst contains an efficiency enhancing salt of a redox-half reaction pair and in which preferably a gaseous member of a redox-half reaction pair is present. In determining the enhancement in efficiency or activity stability, the process and catalyst should be under steady state conditions. In some instances, the catalyst activates over a period of time, even as much as a week or more, before the catalyst reaches peak initial activity. The reason for this period of activation in some catalysts is not known and may be due to chemical and/or physical conditioning of the catalyst. Therefore, the initial activity of a catalyst is usually determined after the catalyst has been on-stream for at least about 24, preferably for at least about 120 to 170, hours.

The optimal amount of the stability enhancing component may vary with the particular stability enhancing component employed, the silver content, the amounts and types of other promoters present and the chemical and physical properties of the carrier. However, the stability enhancing component is often present in an amount of at least 10, preferably at least about 25, ppmw (parts per million by weight) calculated as the weight of the metal in the stability enhancing component on the total catalyst weight. If too much stability enhancing component is used, the catalyst performance, e.g., efficiency and/or activity, may suffer. If too little stability enhancing component is present, it is also possible that the performance of the catalyst will suffer or will be insufficient to show the desired catalytic, e.g., efficiency stabilizing, effect. In determining desired amounts of stability enhancing component, a traverse of stability enhancing component concentrations in the catalyst composition can be effected with the catalysts being evaluated for performance. In some instances, it may be desirable to vary the amounts of other components, e.g., silver and other promoters, to achieve beneficial combinations of effects and optimal catalyst performances. Usually, the amount of stability enhancing component falls within the range of about 25 to 1000, preferably, about 50 to 500, ppmw calculated as the weight of the metal in the stability enhancing component and based on the total catalyst weight.

The stability enhancing component can be provided in various forms, e.g., as a covalent compound such as an oxide, as a cation or as an anion such as a salt, acid or base. The one or more specific species of the stability enhancing component that provide enhanced stability are not certain and may be the component added and/or that generated either during catalyst preparation and/or during use as a catalyst.

Although the species that provide the beneficial properties to the catalysts are not known with specificity, suitable results may be obtained when the stability enhancing component is added to the catalyst in the form of a cation, e.g., as a salt.

Suitable stability enhancing components include, but are not limited to, metal oxides, metal nitrates, metal nitrites, metal sulfates, metal sulfites, metal carboxylates, e.g., acetates, citrates, lactates, oxalates and the like, metal halides, compounds which contain the promoter metal in an anion, metal oxyhalides, metal amine halides, metal complexes and the like.

Specific examples of stability enhancing components include, but are not limited to, ferrous oxide, ferric oxide, ferrous nitrate, ferric nitrate, ferrous nitrite, ferric nitrite, ferrous sulfate, ferric sulfate, ferrous acetate, ferric acetate, ferrous citrate, ferric citrate, ferrous lactate, ferric lactate, ferrous oxalate, potassium ferric oxalate, ferric oxalate, ferrous chloride, ferric chloride, ammonium ferrate, cesium ferrate, potassium ferrate, sodium ferrate, iron (II) complexes, iron (III) complexes, nickel (II) oxide, nickel (III) oxide, nickel nitrate, nickel nitrite, nickel sulfate, nickel sulfite, nickel acetate, nickel citrate, nickel lactate, nickel oxalate, nickel chloride, nickel (II) complexes, nickel (III) complexes, cuprous oxide, cupric oxide, cuprous nitrate, cupric nitrate, cuprous nitrite, cupric nitrite, cuprous sulfate, cupric sulfate, cuprous sulfite, cupric sulfite, cupric acetate, cupric citrate, cupric oxalate, cupric chloride, cuprous chloride, cuprous complexes, cupric complexes, ammoniated ruthenium oxychloride, ruthenium tetroxide, ruthenium complexes, rhodium amine chloride, rhodium trichloride, rhodium sulfate, rhodium sulfite, rhodium complexes, osmium tetroxide, osmium complexes, iridium dichloride, iridium tetrachloride, iridium oxalate, iridium sulfate, iridium complexes, cerous oxide, cerous nitrate, cerous chloride, ceric oxide, ceric nitrate, ceric chloride, cerous complexes, ceric complexes, counterpart compounds for the other lanthanide series rare earths, rhenium oxide, rhenium nitrate, rhenium chloride, potassium perrhenate, cesium perrhenate, rhenium complexes, antimony oxide, antimony nitrate, antimony chloride, antimony complexes, gold oxide, gold nitrate, gold chloride, gold complexes, zinc oxide, zinc nitrate, zinc chloride, zinc complexes, thallium oxide, thallium nitrate, thallium chloride, lead oxide, lead nitrate, lead sulfate, lead chloride, lead complexes, stannous oxide, stannous nitrate, stannous chloride, stannic oxide, stannous nitrate, stannous chloride, stannous complexes, stannic complexes, cadmium oxide, cadmium nitrate, cadmium chloride, cadmium complexes and the like. Examples of complexing agents useful in forming the metal complexes include ethylenediaminetetraacetic acid (EDTA); N, N'-ethylenediaminediacetic acid; N-hydroxyethylethylenediaminetriacetic acid; diethylenetriaminepentaacetic acid; nitrilotriacetic acid; N-hydroxyethyliminodiacetic acid; N-dihydroxyethylglycine; etc... Mixtures of stability enhancing compounds may be used.

The preferred rare earth metals for use in the present invention are cerium, praseodymium, samarium, terbium, neodymium and mixtures thereof.

The stability enhancing components of the present invention preferably comprise at least one of iron, nickel, Copper, ruthenium, rhodium, iridium, rhenium and zinc. More preferably, the stability enhancing component comprises at least one of iron, nickel, copper, rhenium and zinc.

As with any catalyst for making ethylene oxide which provides optimum performance, a correlation exists among many factors. Factors frequently considered include:

(i) the nature of the support;
(ii) the amount of silver on or in the support;
(iii) the components and amounts thereof in or on the support;
(iv) the impurities or contaminants provided with the silver or other components;
(v) the procedure to make the catalyst; and
(vi) the conditions under which the catalyst is used to produce ethylene oxide.

However, in attempting to define any catalyst, there must be a base value from which other factors are determined especially when the factors are variables, each dependent upon the base value for meaning. In the case of this invention, the base value can be the amount of silver or a combination of the amount of silver and the nature of the support. In most cases the latter combination will be the base value. Because at least two values will comprise the base value for catalyst performance, it is apparent that correlations between such combinations and other factors can be quite complex. There is no common thread of logic which integrates all of these combinations and/or factors. To that extent, practice of the invention requires experimental efforts to achieve all or essentially all of the benefits of this invention. Without departing from this script, one skilled in the art can readily achieve the optimum performances of the catalysts of this invention. It should be recognized that such script is commonly followed by the artisan in making any commercially-employable ethylene oxide catalyst. The elements of the script are dependent upon the technology employed in making the catalyst.

The concentration of silver in the finished catalyst may vary from about 2 to 45 or more, often about 2 to 40 or more, weight percent, a commercially preferred range being from about 6% to about 35% by

weight of silver. Lower silver concentrations are preferred from an economic standpoint. However, the optimum silver concentration for any particular catalyst will be dependent upon economic factors as well as performance characteristics, such as catalyst efficiency, rate of catalyst aging and reaction temperature.

The support or carrier employed in these catalysts in its broadest aspects is selected from the large number of porous refractory catalyst carriers or support materials which are considered relatively inert in the presence of the ethylene epoxidation feeds, products and reaction conditions. Many such materials are known to persons skilled in the art and may be of natural or synthetic origin and preferably are of a macroporous structure.

The chemical composition of the carrier is not narrowly critical. Carriers may be composed, for example, of alpha-alumina, silicon carbide, silicon dioxide, zirconia, magnesia and various clays. The preferred carriers are alpha-alumina particles often bonded together by a bonding agent and have a very high purity, i.e., at least 98 wt. % alpha-alumina, any remaining components being silica, alkali metal oxides (e.g., sodium oxide) and trace amounts of other metal-containing and/or non-metal-containing additives or impurities; or they may be of lower purity, e.g., about 80 wt. % alpha-alumina, the balance being a mixture of silicon dioxide, various alkali oxides, alkaline earth oxides, iron oxides, and other metal and non-metal oxides. The carriers are formulated so as to be inert under catalyst preparation and reaction conditions. A wide variety of such carriers are commercially available. Alumina carriers are manufactured by United Catalysts, Inc., Louisville, Kentucky, and the Norton Company, Akron, Ohio.

In the case of alpha alumina-containing supports, preference is given to those having a specific surface area as measured by the B.E.T. method of from about 0.03 $m^2/g$ to about 10 $m^2/g$, preferably from about 0.05 $m_2/g$ to about 5 $m^2/g$, more preferably from about 0.1 $m^2/g$ to about 3 $m^2/g$, and a water pore volume as measured by conventional water absorption techniques of from about 0.1 cc/g to about 0.95 cc/g by volume. The B.E.T. method for determining specific surface area is described in detail in Brunauer, S., Emmet, P. and Teller, E. J. Am. Chem. Soc., 60, 309-16 (1938).

Certain types of alpha alumina-containing supports are particularly preferred. These alpha alumina supports have relatively uniform pore diameters and are more fully characterized by having (1) B.E.T. specific surface areas of from about 0.1 $m^2/g$ to about 3.0 $m^2/g$, preferably about 0.1 $m^2/g$ to about 2.0 $m^2/g$ and (2) water pore volumes of from about 0.10 cc/g to about 0.85 cc/g, preferably from about 0.25 cc/g to about 0.75 cc/g. Median pore diameters for the above-described carriers range from about 0.01 to 100 microns, a more preferred range being from about 0.5 to 50 microns.

The carriers may have monomodal, bimodal or multimodal pore distributions. Typical properties of some supports found in the literature are shown in Table I.

TABLE I

| Carrier | A | B | C | D | E | F |
|---|---|---|---|---|---|---|
| B.E.T. Surface Area $m^2/g$[a] | 0.21 | 0.42 | 0.42 | 0.48 | 0.57 | 2.06 |
| Water Pore Volume, cc/g | 0.26 | 0.36 | 0.41 | 0.49 | 0.44 | 0.65 |
| Crush Strength, FPCS, lbs[b] | 100% 20 lbs | 97% 15 | Avg. 21 Range 15-30 | 90% 14 | 90% 15 | No Data |
| Total Pore Volume, Hg, cc/g[c] | 0.26 | 0.42 | 0.42 | 0.46 | 0.42 | 0.65 |
| Average Pore Diameter, Hg, Angstroms[c] | 620 | 560 | 640 | 550 | 770 | 1000 |
| Median Pore Diameter, Hg, microns [c,e] | 3.7 | 2.7 | 3.4 | 3.4 | 2.4 | 2.5 |
| Percent Pore Volume in Pores Greater than 350 Angstroms[c] | 90.0% | 88.5% | 89.5% | 89.1% | 91.5% | 94.1% |
| Percent Pore Volume in Pores Greater than 1 Micron[c] | 87.0% | 82.5% | 83.4% | 82.3% | 83.5% | 61.0% |
| % Wt. Alpha Alumina | 99.5 | 98 | 98.5 | 98.5 | 98 | 70-75 |
| Water Leachable Na, ppmw | 12 | 53 | 21 | 24 | 18 | No Data |
| Acid-Leachable Na, ppmw | 40 | 96 | 87 | 51 | 45 | No Data |
| Water-Leachable K, ppmw | 5 | 22 | 21 | 22 | 10 | No Data |
| Acid-Leachable Fe, ppmw | 2 | 5 | No Data | 1 | 5 | No Data |
| % Wt. $SiO_2$ | .5 | 2 | 1.5 | 15 | 2 | 25-30 |

(a) Method of Brunauer, Emmet and Teller, loc. cit.
(b) Flat Plate Crush Strength, single pellet.
(c) Determined by mercury intrusion to 55,000 psia using Micrometrics Autopore 9200 or 9210 (130°Contact angle, 0.473 N/m surface tension of Hg).
(e) Median pore diameter represents the pore diameter wherein 50% of the total pore volume is found in pores having less than (or greater than) the median pore diameter.

Regardless of the character of the support or carrier used, it is preferably shaped into particles, chunks, pieces, pellets, rings, spheres, wagon wheels, cross-partitioned hollow cylinders (or rings), and the like of a size suitable for employment in fixed bed reactors. Conventional commercial fixed bed ethylene oxide reactors are typically in the form of a plurality of parallel elongated tubes (in a suitable shell) approximately 0.7 to 2.7 inches O.D. and 0.5 to 2.5 inches I.D. and 15-45 feet long filled with catalyst. In such reactors, it is desirable to employ a support formed into a rounded shape, such as, for example, spheres, pellets, rings, cross-partitioned rings, tablets and the like, having diameters from about 0.1 inch to about 0.8 inch.

As with any supported catalyst, the optimal performance will depend upon optimizing the carrier in terms of its chemical composition (including impurities), surface area, porosity and pore volume. However, the enhancement in performance provided by this invention may be most pronounced when using less than optimized carriers.

The catalysts of this invention contain, in addition to the stability enhancing component or components, at least one other promoter or modifier to enhance the performance of the catalyst, e.g., to enhance efficiency and/or reduce the burning of ethylene oxide and/or affect activity. These promoters or modifiers are generally provided as chemical compounds.

The catalysts of this invention are the type comprising at least one efficiency-enhancing salt of a member of a redox-half reaction pair and preferably are intended to be employed in epoxidation processes in which at least one efficiency-enhancing gaseous member of a redox-half reaction pair is present (described hereinbelow). The term "redox-half reaction" is defined herein to mean half-reactions like those

found in equations presented in tables of standard reduction or oxidation potentials, also known as standard or single electrode potentials, of the type found in, for instance, "Handbook of Chemistry", N. A. Lange, Editor, McGraw-Hill Book Company, Inc., pages 1213-1218 (1961) or "CRC Handbook of Chemistry and Physics", 65th Edition, CRC Press, Inc., Boca Raton, Florida, pages D155-162 (1984). The term "redox-half reaction pair" refers to the pairs of atoms, molecules or ions or mixtures thereof which undergo oxidation or reduction in such half-reaction equations. Such terms as redox-half reaction pairs are used herein to include those members of the class of substances which provide the desired performance enhancement, rather than a mechanism of the chemistry occurring. Preferably, such compounds, when associated with the catalyst as salts of members of a half reaction pair, are salts in which the anions are oxyanions, preferably an oxyanion of a polyvalent atom; that is, the atom of the anion to which oxygen is bonded is capable of existing, when bonded to a dissimilar atom, in different valence states. Potassium is the preferred cation, although sodium, rubidium and cesium may also be operable, and the preferred anions are nitrate, nitrite and other anions capable of undergoing displacement or other chemical reaction and forming nitrate anions under epoxidation conditions. Preferred salts include $KNO_3$ and $KNO_2$, with $KNO_3$ being most preferred.

The salt of a member of a redox-half reaction pair is added in an amount sufficient to enhance the efficiency of the epoxidation reaction. The precise amount will vary depending upon such variables as the gaseous efficiency-enhancing member of a redox-half reaction used and concentration thereof, the concentration of other components in the gas phase, the amount of silver contained in the catalyst, the surface area of the support, the process conditions, e.g., space velocity and temperature, and morphology of support. Generally, however, a suitable range of concentration of the added efficiency-enhancing salt, calculated as cation, is about 0.01 to about 5 percent, preferably about 0.02 to about 3 percent, by weight, based on the total weight of the catalyst. Most preferably the salt is added in an amount of about 0.03 to about 2 weight percent.

One class of promoters includes manganese components. In many instances, manganese components can enhance the activity and/or stability of catalysts. The manganese species that provides the enhanced activity and/or stability is not certain and may be the component added or that generated either during catalyst preparation or during use as a catalyst. Manganese components included, but are not limited to, manganese acetate, manganese ammonium sulfate, manganese citrate, manganese dithionate, manganese oxalate, manganous nitrate, manganous sulfate, and manganate anion, e.g., permanganate anion, manganate anion, manganese complexes, e.g., with manganese in the +2 or +3 oxidation state and complexed with a complexing agent such as a complexing agent selected from those set forth elsewhere herein, and the like. When used, the manganese component is often provided in an amount of at least about 1, say, at least about 5, e.g., about 10 to 2000, often between about 20 and 1000, ppmw calculated as the weight of manganese based on the total weight of the catalyst.

Another class of promoters includes cobalt components. In many instances, cobalt components con enhance the activity and/or efficiency and/or stability of catalysts. The cobalt species that provides the enhanced activity and/or efficiency and/or stability is not certain and may be the component added or that generated either during catalyst preparation or during use as a catalyst. Cobalt components included, but are not limited to, cobaltous oxide, cobaltic oxide, cobaltous nitrate, cobaltic nitrate, cobaltous nitrite, cobaltic nitrite, cobaltous sulfate, cobaltic sulfate, cobaltous acetate, cobaltic acetate, cobaltous citrate, cobaltic citrate, cobaltous lactate, cobaltic lactate, cobaltous oxalate, cobaltic oxalate, cobaltous chloride, cobaltic chloride, ammonium cobaltate, cesium cobaltate, potassium cobaltate, sodium cobaltate, cobalt complexes, e.g., with cobalt in the +2 or +3 oxidation state and complexed with a complexing agent, such as a complexing agent selected from those set forth elsewhere herein, and the like. When used, the cobalt component is often provided in an amount of at least about 10 or 20, e.g., about 25 to 1000, preferably about 50 to 500, ppmw calculated as the weight of cobalt based on the total weight of the catalyst.

In any event, each promoter, such as each metal-containing promoter, e.g., whether cationic, anionic or nonionic, is provided in a promoting amount. As used herein the term "promoting amount" of a certain component of a catalyst refers to an amount of that component that works effectively to provide an improvement or enhancement in one or more of the catalytic properties of that catalyst when compared to a catalyst not containing said component. Examples of catalytic properties include, inter alia, operability (resistance to run-away), selectivity, activity, conversion, stability and yield. It is understood by one skilled in the art that one or more of the individual catalytic properties may be enhanced by the "promoting amount" while other catalytic properties may or may not be enhanced or may even be diminished. Indeed, the promoter may enhance efficiency but decrease activity of the catalyst as determined under Standard Ethylene Oxide Process Conditions. It is further understood that different catalytic properties may be enhanced at different operating conditions. For example, a catalyst having enhanced selectivity at one set of operating conditions may be operated at a different set of conditions wherein the improvement shows up in

the activity rather than the selectivity and an operator of an ethylene oxide plant will intentionally change the operating conditions in order to take advantage of certain catalytic properties even at the expense of other catalytic properties in order to maximize profits by taking into account feedstock costs, energy costs, byproduct removal costs and the like.

The promoting effect provided by the promoters can be affected by a number of variables such as for example, reaction conditions, catalyst preparative techniques, surface area and pore structure and surface chemical properties of the support, the silver and co-promoter content of the catalyst, and the presence of other cations and onions present on the catalyst. The presence of other activators, stabilizers, promoters, enhancers or other catalyst improvers can also affect the promoting effects.

A variety of procedures may be employed for preparing catalysts in accordance with the present invention. The preferred procedure comprises: (1) impregnating a porous catalyst carrier with a solution comprising a solvent or solubilizing agent, silver complex in an amount sufficient to deposit the desired weight of silver and the aforementioned stability enhancing component or components upon the carrier, and (2) thereafter treating the impregnated support to convert the silver salt to silver metal and effect deposition of silver and the stability enhancing component or components onto the exterior and interior surfaces of the support. For sake of repeatability, in the use and reuse of impregnating solutions the carrier should preferably not contain undue amounts of ions which are soluble in the impregnating solution and/or exchangeable with the stability enhancing component or components supplied to the catalyst, either in the preparation or use of the catalyst, so as to upset the amount of stability enhancing component or components which provide the desired catalyst enhancement. If the carrier contains such ions, the ions should generally be removed by standard chemical techniques such as leaching. Silver and stability enhancing component depositions are generally accomplished by heating the carrier at elevated temperatures to evaporate the remaining liquid within the support and effect deposition of the silver and stability enhancing component or components onto the interior and exterior carrier surfaces. Impregnation of the carrier is the preferred technique for silver deposition because it utilizes silver more efficiently than coating procedures, the latter being generally unable to effect substantial silver deposition onto the interior surface of the carrier. In addition, coated catalysts are more susceptible to silver loss by mechanical abrasion.

The sequence of impregnating or depositing the surfaces of the carrier with silver and stability enhancing component or components is optional. Thus, impregnation and deposition of silver and salts may be effected coincidentally or sequentially, i.e., the stability enhancing component or components may be deposited prior to, during, or subsequent to silver addition to the carrier. The stability enhancing component or components and the other promoters may be deposited together or sequentially. For example, one or more of the salts may be deposited first followed by the coincidental or sequential deposition of silver and additional or other salts. In instances in which the silver component in the silver impregnation solution may interfere with the compound providing the stability enhancing component, or, alternatively, the compound providing the stability enhancing component may interfere with the silver component in the impregnation solution, it is frequently desirable to use a sequential deposition process in which the silver is deposited and calcined and then the stability enhancing component compound is impregnated onto the support.

Impregnation of the catalyst carrier is effected using one or more solutions containing silver, stability enhancing components and other promoters in accordance with well-known procedures for coincidental or sequential depositions. For coincidental deposition, following impregnation the impregnated carrier is heat or chemically treated to reduce the silver compound to silver metal and deposit the silver, stability enhancing components and other promoters onto the catalyst surfaces.

For sequential deposition, the carrier is initially impregnated with silver or one or more stability enhancing components or one or more other promoters (depending upon the sequence employed) and then heat or chemically treated as described above. This is followed by one or more additional impregnation steps and corresponding heat or chemical treatments to produce the finished catalyst containing silver and the stability enhancing component or components and other promoter or promoters.

In one useful embodiment, the silver-containing carrier is subsequently impregnated using a solution containing a metal-containing promoter other than alkali metals, or alkaline earth metals, the solution being chosen so that the metal-containing promoter has an increased affinity to the silver-containing carrier relative to its affinity to the carrier without silver and/or is associated with the silver-containing carrier at an increased rate relative to the rate at which the promoter would be associated with the carrier without silver. This method facilitates catalyst preparation and produces catalysts which are effective for alkene epoxidation. This method is more fully described in commonly assigned U.S. patent application Serial No. (Attorney's Docket No. D-16588, filed ________, which is incorporated in its entirety herein by reference.

In making the catalysts of this invention, some promoters such as some alkali and alkaline earth metal salts have such high melting temperatures that when deposited on the support with silver compound, and

subjected to heating to convert the silver compound to silver metal, the salts may remain essentially unchanged. Of course, it is realized that alkali metal and alkaline earth metal salts having an unstable oxidation state will change to a stable oxidation state or states, e.g., sulfites to sulfates. When, for instance, the alkali metal or alkaline earth metal is deposited as the hydroxide or carbonate, it may be transformed in the presence of amines, which may be used in the impregnation of the catalyst, to a different salt form (i.e., nitrate) during the heating (roasting) step depending on the roast conditions.

The silver solution used to impregnate the carrier is comprised of a silver compound in a solvent or complexing/solubilizing agent such as the silver solutions disclosed in the art. The particular silver compound employed may be chosen, for example, from among silver complexes, nitrate, silver oxide or silver carboxylates, such as silver acetate, oxalate, citrate, phthalate, lactate, propionate, butyrate and higher fatty acid salts. Desirably, silver oxide complexed with amines is the preferred form of silver in the practice of the invention.

A wide variety of solvents or complexing/solubilizing agents may be employed to solubilize silver to the desired concentration in the impregnating medium. Among those disclosed in the art as being suitable for this purpose are lactic acid (U.S. Patent Nos. 2,477,436 to Aries; and 3,501,417 to DeMaio); ammonia (U.S. Patent 2,463,228 to West, et al.); alcohols, such as ethylene glycol (U.S. Patent Nos. 2,825,701 to Endler, et al.; and 3,563,914 to Wattimina); and amines and aqueous mixtures of amines (U.S. Patent Nos. 2,459,896 to Schwarz; 3,563,914 to Wattimina; 3,215,750 to Benisi; 3,702,259 to Nielsen; and 4,097,414, 4,374,260 and 4,321,206 to Cavitt).

The metal-containing promoter or promoters, meaning to include the presently useful stability enhancing component or components as well as other metal-containing promoter or promoters, themselves may be present as complexes in the impregnating solution, in particular in such a solution which also contains silver, prior to being associated with the carrier. Such complexes may conveniently be derived by including one or more complexing agents effective to form a complex with at least one metal species, e.g., metal-containing promoter precursor, in the impregnating solution, or solution precursor (e.g., a liquid medium containing undissolved metal-containing promoter precursor) in an amount effective to enhance the solubility and/or solubility stability of the metal-containing promoter in the solution or solution precursor. The enhancement in solubility and/or solubility stability is determined by comparing similar impregnating solutions or solution precursors in which the metal-containing promoter is complexed and in which the metal-containing promoter is not complexed with the complexing agent and/or with and without the complexing agent. The term "solubility stability" is a measure of the ability of a metal-containing promoter to remain in solution over time, the longer the time the more solubility stable the metal-containing promoter is.

The complexing agent or agents useful to form the metal-containing promoter complexes thereof may be chosen, e.g., from among conventional and well known complexing agents, to provide the desired solubility and/or solubility stability. The selection of the particular complexing agent or agents to be employed is dependent on many factors, such as, the metal-containing promoter to be employed, the composition of the impregnation solution or solution precursor, the conditions at which the impregnation solution or solution precursor is to be held prior to being used to impregnate the carrier, etc. Examples of complexing agents which may be useful include ethylenediaminetetraacetic acid (EDTA); N, N'-ethylenediaminediacetic acid; N-hydroxyethylethylenediaminetriacetic acid; diethylenetriaminepentaacetic acid; nitrilotriacetic acid; N-hydroxyethyliminodiacetic acid; N-dihydroxyethylglycine; etc...

The amount of complexing agent employed varies widely, for example, depending on the specific complexing agent and on the specific metal species to be complexed, and on the amount of metal species to be complexed. Preferably, the amount of complexing agent is at least about 50%, more preferably at least about 100%, of that needed to form complexes with the metal species to be complexed in the impregnating solution or solution precursor. Excesses of complexing agent over that needed to form the desired complexes may be employed, for example, so that the complexes can be maintained over a relatively long period of time. For example, the complexing agent may be included in an amount of at least about 150% or at least about 200% or at least about 400% or more of that needed to form the desired complexes. The amount of complexing agent employed, e.g., in the impregnating solution or solution precursor, includes both the complexing agent which is complexed with the metal species and the additional or excess complexing agent, if any, which is present in the impregnating solution or solution precursor and is not so complexed.

The use of complexing agents to increase the solubility stability of impregnating mixtures, e.g., solutions, may be employed with a wide variety of metal-containing promoters useful in silver-containing alkene epoxidation catalysts. For example, complexing agents can be used in impregnating carriers with promoters which include metals of Group 1b, 2b, 3b, 4b, 5b, 6b, 7b and 8 of the Periodic Table, the rare earth metals, tin, antimony, lead, thallium and bismuth and mixtures thereof. (References to the Periodic

Table herein shall be to that as published by the Chemical Rubber Company, Cleveland, Ohio, In CRC Handbook of Chemistry and Physics, 46th Edition, inside back cover.) The use of complexing agents is particularly useful in impregnating metal-containing promoters including manganese, iron, cobalt, nickel, copper, gold, ruthenium, rhodium, osmium, iridium, zinc, cadmium, the rare earth metals, rhenium, antimony, gold, zinc, thallium, lead and mixtures thereof, especially manganese, cobalt and mixtures thereof. The use of complexing agents, provides particularly beneficial results when solutions without such complexing agents, such as manganese-containing solutions without such complexing agents, tend to relatively quickly become unstable and form precipitates. Unless such solutions without complexing agents are used promptly after they are formed, the resulting catalyst may be non-uniform and may have less than acceptable catalytic properties. Also, the constraint of having to use an impregnation solution promptly after formation reduces processing flexibility and may increase costs. The use of complexing agents, as described herein, mitigates against this constraint and provides effective alkene epoxidation catalysts.

A particularly preferred process for making high silver content catalysts involves two or more sequential impregnations of silver, with or without promoters, each of which impregnations may be followed by roasting or other procedure to render the silver insoluble. Advantageously, the carrier has a high pore volume and surface area when using high silver loadings.

Following impregnation of the catalyst carrier with silver and/or the stability enhancing component or components and/or the other promoter or promoters, the impregnated carrier particles are separated from any remaining non-absorbed solution. This is conveniently accomplished by draining the excess impregnating medium or, alternatively, by using separation techniques, such as filtration or centrifugation. The impregnated carrier is then generally heat treated (e.g., roasted) to effect decomposition and reduction of the silver metal compound (complexes in most cases) to metallic silver and/or the deposition of the stability enhancing component or components and/or the other promoter or promoters. Such roasting may be carried out at a temperature of from about 100°C to 900°C, preferably from about 200°C to 700°C, for a period of time sufficient to convert substantially all of the silver salt to silver metal and/or to effect deposition of substantially all the stability enhancing component or components and/or the other promoters. In general, the higher the temperature, the shorter the required time period. For example, at a temperature of from about 400°C to 900°C, silver reduction may be accomplished in about 1 to 5 minutes. Although a wide range of heating periods have been suggested in the art to thermally treat the impregnated support, (e.g., U.S. Patent 3,563,914 suggests heating for less than 300 seconds to dry, but not roast to reduce, the catalyst; U.S. Patent 3,702,259 discloses heating from 2 to 8 hours at a temperature of from 100°C to 375°C to reduce the silver salt in the catalyst; and U.S. Patent 3,962,136 suggests 1/2 to 8 hours for the same temperature range), it is only important that the time be correlated with temperature such that substantially complete reduction of the silver salt to metal and/or substantially complete deposition of the stability enhancing component or components and/or other promoter or promoters accomplished. A continuous or step-wise heating program is desirably used for this purpose. Continuous roasting of the catalyst for a short period of time, such as for not longer than 1/2 hour is preferred and can be effectively done in making the catalysts of this invention.

Heat treatment is preferably carried out in air, but a nitrogen or carbon dioxide atmosphere may also be employed. The equipment used for such heat treatment may use a static or flowing atmosphere of such gases to effect reduction, but a flowing atmosphere is much preferred.

An important consideration in making the catalyst of this invention is to avoid the use of strongly acidic or basic solutions which can attack the support and deposit impurities which can adversely affect the performance of the catalyst. The preferred impregnation procedure of U.K. Patent 2,043,481 coupled with the high roasting temperature, short residence time procedure which the patent also described is especially beneficial in minimizing such catalyst contamination. However, the use of the salts of this invention coupled with the high purity supports allows one to use lower temperatures though short residence times are preferred.

The particle size of silver metal deposited upon the carrier is asserted by a portion of the prior art to be a function of the catalyst preparation procedure employed. This may seem to be the case because of the limited ability of the art to effectively view the surface of the catalyst. Thus the space between the silver particles seen on the carrier has not been characterized sufficiently to say whether such particles of silver represent all the silver on the carrier. However, the particular choice of solvent and/or complexing agent, silver compound, heat treatment conditions and catalyst carrier may affect, to varying degrees, the range of the size of the resulting silver particles seen on the carrier. For carriers of general interest for the production of ethylene oxide, a distribution of silver particle sizes in the range of 0.005 to 2.0 microns is typically obtained. However, the role of particle size of the silver catalyst upon the effectiveness of the catalyst in making ethylene oxide is not clearly understood. In view of the fact that the silver particles are known to

migrate on the surface of the catalyst when used in the catalytic reaction resulting in a marked change in their size and shape while the catalyst is still highly effective suggests that the silver particle size viewed on the support may not be a significant factor in catalytic performance.

The silver catalysts of the invention are particularly suitable for use in the production of ethylene oxide by the vapor phase oxidation of ethylene with molecular oxygen. The reaction conditions for carrying out the oxidation reaction are well-known and extensively described in the prior art. This applies to reaction conditions, such as temperature, pressure, residence time, concentration of reactants, gas phase diluents (e.g., nitrogen, methane and $CO_2$), gas phase inhibitors (e.g., ethylene chloride and ethylene dichloride), and the like.

The gases fed to the reactor preferably contain at least one gaseous efficiency-enhancing member of a redox-half reaction pair.

The terms "gaseous member of a redox-half reaction pair", "gaseous efficiency-enhancing member of a redox-half reaction pair", or like terms referred to herein have a meaning similar to that for the "salt of a member of a redox-half reaction pair" or like terms, defined above. That is, these terms refer to members of half-reactions, represented in standard or single electrode potential tables in standard reference texts or handbooks which are in a gaseous state and are substances which, in the reaction equations represented in the texts, are either oxidized or reduced. The preferred gaseous efficiency-enhancing materials are compounds containing an element capable of existing in more than two valence states, preferably nitrogen and another element which is, preferably, oxygen. Examples of preferred gaseous efficiency-enhancing members of redox-half reaction pairs include at least one of NO, $NO_2$, $N_2O_3$, $N_2O_4$,$N_2O_5$ or any gaseous substance capable of forming one of the aforementioned gases, particularly NO and $NO_2$, under epoxidation conditions, and mixtures thereof with one or more of $PH_3$, CO, $SO_3$, $SO_2$, $P_2O_5$, and $P_2O_3$. NO is often preferred as the gaseous efficiency-enhancing compound.

Although in some cases it is preferred to employ members of the same half-reaction pair in the reaction system, i.e., both the efficiency-enhancing salt member associated with the catalyst and the gaseous member in the feedstream, as, for example, with a preferred combination of potassium nitrate and nitric oxide, this is not necessary in all cases to achieve satisfactory results. Other combinations, such as $KNO_3/N_2O_3$, $KNO_3/NO_2$, $KNO_3/N_2O_4$, $KNO_3/SO_2$, $KNO_2/NO$, $KNO_2/NO_2$ and $KNO_3$/a mixture of $SO_2$ and NO, may also be employed in the same system. In some instances, the salt and gaseous members may be found in different half-reactions which represent the first and last reactions in a series of half-reaction equations of an overall reaction.

The gaseous efficiency-enhancing member of a redox-half reaction pair is also present in an amount sufficient to enhance the performance, such as the activity of the catalyst, and, particularly, the efficiency of the epoxidation reaction. The precise amount is determined, in part, by the particular efficiency-enhancing salt of a member of a redox-half reaction pair used and the concentration thereof, the particular alkene undergoing oxidation, and by other factors noted above which influence the amount of efficiency-enhancing salt of a member of a redox-half reaction pair. Typically a suitable concentration of the gaseous member of a redox-half reaction pair for epoxidation of most alkenes, including propylene, is about 0.1 to about 2,000 ppm, by volume, of the gaseous feedstream when $N_2$ is used as ballast. When a preferred gaseous member of a redox-half reaction pair, such as NO, is used in the epoxidation of propylene, the preferred concentration is about 2,000 ppm, by volume, with an $N_2$ ballast. However, when ethylene is being oxidized, a suitable concentration is from about 0.1 to about 100 ppm, by volume, of the gaseous feedstream components. Preferably, the gaseous efficiency-enhancing member of a redox-half reaction pair is present in an amount of about 1 to about 80 ppm when about 3 percent, by volume, $CO_2$ is present in the reaction mixture. When nitric oxide is employed as the gaseous efficiency-enhancing compound in an ethylene epoxidation system, it is present in an amount of about 0.1 to about 60 ppm, preferably about 1 to about 40 ppm, when $CO_2$ is present in the reaction mixture, e.g., in amounts up to about 3 volume percent.

The desirability of recycling unreacted feed, or employing a single-pass system, or using successive reactions to increase ethylene conversion by employing reactors in series arrangement can be readily determined by those skilled in the art. The particular mode of operation selected will usually be dictated by process economics.

Generally, the commercially-practiced processes are carried out by continuously introducing a feed stream containing ethylene and oxygen to a catalyst-containing reactor at a temperature of from about 200°C to 300°C, and a pressure which may vary from about five atmospheres to about 30 atmospheres depending upon the mass velocity and productivity desired. Residence times in large-scale reactors are generally on the order of about 0.1-5 seconds. Oxygen may be supplied to the reaction in an oxygen-containing stream, such as air or as commercial oxygen. The resulting ethylene oxide is separated and recovered from the reaction products using conventional methods. However, for this invention, the ethylene

oxide process envisions the normal gas recycle encompassing carbon dioxide recycle in the normal concentrations, e.g., about 0.5 to 6 volume percent.

Catalysts which contain one or more of the present stability-enhancing components, manganese components, cobalt components and mixtures thereof, hereinafter referred to as Stability Catalysts, may be used selectively to enhance the effectiveness of an alkene epoxidation process, as described herein. For example, as noted previously, conventional silver-containing ethylene epoxidation catalysts are often subjected to reaction conditions for relatively long periods of time, e.g., on the order of one week or more, before achieving the desired high catalyst activity. This, "slow start-up" phenomenon is particularly apparent in the fixed catalyst bed, recycle-type of operation often used commercially. The present Stability Catalyst is effective to increase or enhance the rate of start-up of the alkene epoxidation process. That is, the present Stability Catalyst achieves the desired high catalyst activity, e.g., on alkene epoxidation process start-up, at a faster rate or in a shorter period of time relative to a similar catalyst which does not contain the stability enhancing component, manganese component or cobalt component, e.g., at CONDITIONS. This feature is beneficial, for example, by providing for increased process efficiency and reduced costs.

An additional feature of the present Stability Catalyst is its increased ability to maintain catalytic effectiveness, e.g., in terms of activity and/or efficiency and/or stability, from the front end or inlet of the fixed catalyst bed through the fixed catalyst bed to the back end or outlet of the fixed catalyst bed. Similar alkene epoxidation catalysts which do not include the stability-enhancing component, manganese component or cobalt component tend to be more effective at or near the front end of a fixed catalyst bed, with catalyst effectiveness deteriorating for the catalyst away from the front end and at or near the back end of the fixed catalyst bed. The present Stability Catalyst mitigates against this deterioration. That is, the present Stability Catalyst provides increased catalyst effectiveness stability along the length of a fixed bed of catalyst relative to a similar alkene epoxidation catalyst which includes no stability-enhancing component, manganese component or cobalt component. Thus, the present Stability Catalyst provides for increased overall catalyst effectiveness.

Although the reason or reasons why the present Stability Catalyst provides faster alkene epoxidation process start-up rates and mitigate against catalyst deterioration along a fixed catalyst bed are not fully understood, a number of process modifications can be employed to take advantage of these catalyst characteristics. For example, the fixed catalyst bed can be graded in stability-enhancing component, manganese component and/or cobalt component concentration from the front end to the back end, e.g., with the catalyst at or near the front end of the catalyst bed containing the highest concentration of stability-enhancing component, manganese component and/or cobalt component and the catalyst at or near the back end of the catalyst bed containing the lowest concentration of stability-enhancing component, manganese component and/or cobalt component. Alternatively, one or more portions of the fixed catalyst bed may contain Stability Catalyst while one or more other portions of the same fixed catalyst bed contain non-Stability Catalyst. Preferably, the catalyst at or near the front end of the catalyst bed is Stability Catalyst. A single fixed catalyst bed may be divided into two or more segments to facilitate the inclusion of different catalysts therein.

A still further processing modification is to treat, e.g., pre-treat, one or more of the gaseous components, or a portion of all of the gaseous stream, fed to the alkene epoxidation reaction system with a Stability Catalyst to condition such component or components for alkene epoxidation. As used in this context, the term "condition" refers to a treatment of the gaseous component or components, or a portion or all of the gaseous stream, which is effective to provide an increased rate of alkene epoxidation process start-up and/or increased catalyst effectiveness along the length of the fixed catalyst bed relative to using untreated gaseous component or components, or an untreated portion or all of the gaseous stream, e.g., at CONDITIONS. The entire fresh feedstream and/or the entire recycle stream, preferably both the fresh feedstream and the entire recycle stream, may be contacted with a Stability Catalyst, e.g., at alkene epoxidation conditions, to provide such conditioning. After such conditioning, alkene epoxidation can be conducted using the conditioned component or components with a Stability Catalyst, a non-Stability Catalyst or a combination of Stability and non-Stability Catalysts.

In commercial processes, typical operating conditions can vary and the amounts of the ingredients employed can be adjusted to achieve the best efficiencies. In particular the amounts of ethane, carbon dioxide, organic chloride, nitrogen oxide can be varied to optimize efficiency for the manufacture of ethylene oxide. Ethane is an impurity contained in varying amounts in ethylene raw material. Ethane can also be added to a commercial reactor to provide better control of the chloride's inhibitor action. Typically, the amount of ethane used in commercial processes can vary from about 0.001 to about 5 mole percent for achieving optimization under both air process conditions and oxygen process conditions. As the concentration of ethane increases in the reactor, the effective surface chloride concentration on the catalyst is

believed to be decreased thereby decreasing the ability of chloride to promote/inhibit reactions that increase efficiency for the manufacture of ethylene oxide. The amount of chloride, e.g., ethyl chloride or ethylene dichloride, can be varied to provide the needed promoter/inhibitor action commensurate with the ethane levels encountered in a particular process and the type of promoters or modifiers used in the catalyst. The amount of organic chloride used in commercial processes can typically vary from about 1.0 ppm to about 100 ppm for achieving optimization under both air process conditions and oxygen process conditions. Carbon dioxide is generally considered an inhibitor, and the inhibitor effect of carbon dioxide on process efficiency may be variable with its concentration. With different types of promoters or modifiers used in preparation of the catalysts of this invention, different concentrations of carbon dioxide may be more desirable in certain commercial processes. Typically, the amount of carbon dioxide used in commercial processes can vary from about 2 to about 15 mole percent for achieving optimization under both air process conditions and oxygen process conditions. The amount of carbon dioxide is dependent on the size and type of carbon dioxide scrubbing system employed. The optimization of the amounts of ethane, carbon dioxide and organic chloride provides catalysts which are especially suitable for obtaining desired efficiencies in commercial ethylene oxide manufacture. Especially in the epoxidation processes using at least one gaseous efficiency-enhancing member of a redox-half reaction pair in conjunction with at least one salt of a member of a redox-half reaction pair on the catalyst, the concentration of carbon dioxide is preferably maintained below about 1.5, e.g., below about 1.0 or even about 0.5, volume percent.

Catalysts which have been subjected to process conditions for ethylene oxide manufacture such as STANDARD ETHYLENE OXIDE PROCESS CONDITIONS are considered an important aspect of this invention.

## Examples

The following examples are by way of illustration only and are not to be construed as limiting the scope of the invention described herein.

## CARRIERS "A" AND "B"

Both Carriers A and B are alpha aluminas prepared from a boehmite by fluoride mineralization. Each of these carriers are in the form of simple rings and have the following characteristics:

| | Carrier A | Carrier B |
|---|---|---|
| Surface Area, $m^2/g$(1) | 1.24 | 1.10 |
| Water Pore Volume,% | 77.0 | 86.0 |
| Residual Fluoride, Wt.% | - | 0.12 |
| Water Leachable, ppmw | | |
| Phosphate | 0 | - |
| Fluoride | 36 | - |
| Aluminum | 6 | - |
| Calcium | 9 | - |
| Potassium | 1 | - |
| Magnesium | 5 | - |
| Sodium | 13 | - |
| Silicon | 0 | - |

(1) Method of Measurement described in "Adsorption Surface Area and Porsity", S.J. Gregg and K.S.W. Sing, Academic Press (1967), pages 316-321.

The identity and amounts of water leachable components of carriers can be determined by any

convenient analytical technique. Generally, the carriers are heated in distilled water at a temperature of about 50° to 95°C, often 90°C, for about 0.5 to 2, e.g., 1 hour. The liquid is then subjected to ion chromatography and Inductively Coupled Plasma Spectroscopy techniques.

EXAMPLE 1 (COMPARATIVE)

The preparation technique for the catalyst used in Example 1 is as follows. An impregnation solution is prepared by mixing 11.47 weight parts of ethylenediamine (high purity grade) with 20.00 weight parts of distilled water. Then 11.60 weight parts of oxalic acid dihydrate (reagent grade) are slowly added to the mixture at ambient conditions. The addition of the oxalic acid dihydrate is at a rate that the exotherm does not cause the temperature of the solution to rise above 40°C. Then 19.82 weight parts of silver oxide are added followed by 4.01 weight parts of monoethanolamine (Fe and Cl free). Distilled water is then added to adjust the solution weight to 70.00 weight parts.

About 132 milliliters of this impregnation solution are used for a first impregnation of Carrier A.

Approximately 61.1 grams of Carrier A are placed in an impregnation vessel which is evacuated to about 30 inches-Hg absolute at ambient temperature. 132 milliliters of the impregnation solution is placed in the impregnation vessel under vacuum and allowed to contact the carrier for about 30 minutes. The vessel is then opened and the impregnation solution was drained from the carrier.

The first impregnated carrier is then roasted in hot air using a belt roaster. The catalyst is spread out in a single layer on a 2 5/8 inches wide endless stainless steel belt (spiral weave) and transported through a 2 inches by 2 inches square heating zone for 2.5 minutes. Hot air, heated externally by a tubular furnace, is discharged from a 2 inches by 2 inches discharge port immediately below the belt at about 500°C at a rate of 66 standard cubic feet per hour per square inch.

Another 130 milliliters of the impregnation solution are used for a second impregnation. About 1.125 grams of crystalline $KNO_3$ is added.

The previously impregnated carrier is placed in an impregnation vessel which was evacuated to about 30 inches-Hg absolute at ambient temperature. All the 130 milliliters of the second impregnation solution are placed in the impregnation vessel under vacuum and allowed to contact the carrier for about 15 minutes. The vessel is then opened an the impregnation solution is drained from the carrier.

The second impregnated carrier is then roasted in hot air using a belt roaster. The second impregnated carrier is spread out in a single layer on a 2 5/8 inches wide endless stainless steel belt (spiral weave) and transported through a 2 inches by 2 inches square heating zone for 2.5 minutes. Hot air, heated externally by a tubular furnace, is discharged from a 2 inches by 2 inches discharge port immediately below the belt a about 500° C at a rate of 66 standard cubic feet per hour per square inch.

The catalyst product is analyzed to contain 35.3 weight percent silver and 1405 parts per million by weight of potassium.

The performance of this catalyst, catalyst 1, is determined using a backmix autoclave as described above. Approximately 62.8 grams of catalyst 1 are charged to the autoclave. The initial feed composition is as follows:

oxygen 8 volume percent
ethane 0 volume percent
ethyl chloride 5 parts per million by volume
ethylene 30 volume percent
carbon dioxide 0 volume percent
nitric oxide 5 parts per million by volume

The gas hourly space velocity is 8000 reciprocal hours and the pressure is 275 pounds per square inch gauge. The amounts of nitric oxide and ethyl chloride are adjusted periodically to optimize catalyst activity and efficiency. The performance of catalyst 1 is summarized in Table 2.

EXAMPLE 2

The preparation technique for the catalyst used in Example 2 is as follows. An impregnation solution is prepared by mixing 11.43 weight parts of ethylenediamine (high purity grade) with 24.00 weight parts of distilled water. Then 11.60 weight parts of oxalic acid dihydrate (reagent grade) are slowly added to the mixture at ambient conditions. The addition of the oxalic acid dihydrate is at a rate that the exotherm does not cause the temperature of the solution to rise above 40°C. Then 19.82 weight parts of silver oxide are added followed by 4.00 weight parts of monoethanolamine (Fe and Cl free). Distilled water is then added to adjust the solution weight to 75.00 weight parts.

About 136 milliliters of this impregnation solution are used for a first impregnation of Carrier A.

Approximately 61.5 grams of Carrier A are placed in an impregnation vessel which is evacuated to about 30 inches-Hg absolute at ambient temperature. 136 milliliters of the impregnation solution are placed in the impregnation vessel under vacuum and allowed to contact the carrier for about 30 minutes. The vessel is then opened and the impregnation solution was drained from the carrier.

The first impregnated carrier is then roasted as described in Example 1.

Another 126 milliliters of the impregnation solution are used for a second impregnation. About 1.018 gram of crystalline $KNO_3$ are added directly to the solution and 0.282 grams of $Fe(NO_3)_3.9H_2O$ in 2 ml water are added.

The previously impregnated carrier is placed in an impregnation vessel which was evacuated to about 30 inches-Hg absolute at ambient temperature. All the 128 milliliters of the iron-containing impregnation mixture are placed promptly after iron addition in the impregnation vessel under vacuum and allowed to contact the carrier for about 15 minutes. The vessel is then opened and the impregnation mixture is drained from the carrier.

The second impregnated carrier is then roasted as described in Example 1.

The catalyst product is analyzed to contain 33.3 weight percent silver, 1444 parts per million by weight of potassium nd 134 parts per million by weight of iron.

The performance of this catalyst, catalyst 2, is determined using a 2-inch backmix autoclave as described above. Approximately 61.5 grams of catalyst 2 are charged to the autoclave. The initial feed composition and reaction conditions are as described in Example 1. The performance of catalyst 2 is summarized in Table 2.

## EXAMPLE 3

The preparation technique for the catalyst used in Example 3 is as follows. The first impregnation and roasting are as described in Example 2 except that 128 milliliters of impregnation solution is used to impregnate 61.1 grams of Carrier A.

Another 130 milliliters of the impregnation solution are used for a second impregnation. About 1.026 gram of crystalline $KNO_3$ are added directly to this solution and 0.567 grams of $Fe(NO_3)_3.9H_2O$ in 2 ml water are added.

The previously impregnated carrier is placed in an impregnation vessel which was evacuated to about 30 inches-Hg absolute at ambient temperature. All the 132 milliliters of the iron-containing impregnation mixture are placed promptly after iron addition in the impregnation vessel under vacuum and allowed to contact the carrier for about 15 minutes. The vessel is then opened and the impregnation mixture is drained from the carrier.

The second impregnated carrier is then roasted as described in Example 1.

The catalyst product is analyzed to contain 34.1 weight percent silver, 1434 parts per million by weight of potassium and 266 parts per million by weight of iron.

The performance of this catalyst, catalyst 3, is determined using a 2-inch backmix autoclave as described above. Approximately 62.0 grams of catalyst 3 are charged to the autoclave. The initial feed composition and reaction conditions are as described in Example 1. The performance of catalyst 3 is summarized in Table 2.

## EXAMPLE 4

The preparation technique for the catalyst used in Example 4 is as follows. The first impregnation and roasting are as described in Example 2 except that 125 milliliters of impregnation solution is used to impregnate 61.7 grams of Carrier A.

Another 130 milliliters of the impregnation solution are used for a second impregnation. About 1.027 gram of crystalline $KNO_3$ are added directly to this solution and 0.198 grams of $Ni(NO_3)_2$in 5 ml water are added.

The previously impregnated carrier is placed in an impregnation vessel which was evacuated to about 30 inches-Hg absolute at ambient temperature. All the 135 milliliters of the nickel-containing impregnation mixture are placed in the impregnation vessel under vacuum and allowed to contact the carrier for about 15 minutes. The vessel is then opened an the impregnation mixture is drained from the carrier.

The second impregnated carrier is then roasted as described in Example 1.

The catalyst product is analyzed to contain 33.9 weight percent silver, 1464 parts per million by weight of potassium and 151 parts per million by weight of nickel.

The performance of this catalyst, catalyst 4, is determined using a 2-inch backmix autoclave as described above. Approximately 62.6 grams of catalyst 4 are charged to the autoclave. The initial feed composition and reaction conditions are as described in Example 1. The performance of catalyst 4 is summarized in Table 2.

EXAMPLE 5

The preparation technique for the catalyst used in Example 5 is as follows. The first impregnation and roasting are described in Example 2 except that 125 milliliters of impregnation solution are used to impregnate 52.1 grams of Carrier A.

Another 125 milliliters of the impregnation solution are used for a second impregnation. About 4.421 gram of an aqueous solution containing 0.0696 gram of $KNO_3$ per gram solution and 0.1757 grams of an aqueous solution containing 0.0531 gram of KOH per gram solution and 0.0478 grams of $NH_4ReO_4$ are added.

The previously impregnated carrier is placed in an impregnation vessel which was evacuated to about 30 inches-Hg absolute at ambient temperature. All the 130 milliliters of the rhenium-containing impregnation mixture are placed in the impregnation vessel under vacuum and allowed to contact the carrier for about 15 minutes. The vessel is then opened an the impregnation mixture is drained from the carrier.

The second impregnated carrier is then roasted as described in Example 1.

The catalyst product is analyzed to contain 35.4 weight percent silver and 1330 parts per million by weight of potassium and 120 parts per million by weight of rhenium.

The performance of this catalyst, catalyst 5, is determined using a 2-inch backmix autoclave as described above. Approximately 65.8 grams of catalyst 5 are charged to the autoclave. The initial feed composition and reaction conditions are as described in Example 1. The performance of catalyst 5 is summarized in Table 2.

EXAMPLE 6 (COMPARATIVE)

The preparation technique for the catalyst used in Example 6 is as follows. An impregnation solution is prepared by mixing 22.25 grams of ethylenediamine (high purity grade) with 22.05 grams of distilled water. Then 22.29 grams of oxalic acid dihydrate (reagent grade) are slowly added to the mixture at ambient conditions. The addition of the oxalic acid dihydrate is at a rate that the exotherm does not cause the temperature of the solution to rise above 40°C. Then 39.04 grams of silver oxide are added followed by 7.81 of monoethanolamine (Fe and Cl free). About 0.470 grams of $KNO_3$ in 10 ml water is added. Distilled water is then added to adjust the solution volume to 125 milliliters.

Approximately 47.7 grams of Carrier B are placed in an impregnation vessel which is evacuated to about 28 inches-Hg absolute at ambient temperature. All of the impregnation solution is placed in the impregnation vessel under vacuum and allowed to contact the carrier for about one hour. The vessel is then opened and the impregnation solution was drained from the carrier.

The first impregnated carrier is then roasted as described in Example 1.

The catalyst product is analyzed to contain 19.5 weight percent silver and 974 parts per million by weight of potassium.

The performance of this catalyst, catalyst 6, is determined using a 2-inch backmix autoclave as described above. Approximately 45.8 grams of catalyst 6 are charged to the autoclave. The feed composition is as follows:
oxygen 8 volume percent
ethane 0.3 volume percent
ethyl chloride 20 parts per million by volume
ethylene 30 volume percent
carbon dioxide 3 volume percent
nitric oxide 15 parts per million by volume

The gas hourly space velocity is 8000 reciprocal hours and the pressure is 275 pounds per square inch gauge. The concentrations of nitric oxide and ethyl chloride are periodically adjusted to optimize catalyst activity and efficiency. The performance of catalyst 6 is summarized in Table 2.

EXAMPLE 7

The preparation technique for catalyst used in Example 7 is as follows. An impregnation solution is

prepared by mixing 22.33 grams of ethylenediamine (high purity grade) with 22.13 grams of distilled water. Then 22.36 grams of oxalic acid dihydrate (reagent grade) are slowly added to the mixture at ambient conditions. The addition of the oxalic acid dihydrate is at a rate that the exotherm does not cause the temperature of the solution to rise above 40°C. Then 39.17 grams of silver oxide are added followed by 7.83 of monoethanolamine (Fe and Cl free). About 0.472 gram of $KNO_3$ in 10 ml water and 1.047 grams of $Zn(NO_3)_2$ in 10 ml water are added. Distilled water is then added to adjust the solution weight to 125 milliliters. All of this impregnation solution is used for the impregnation of catalyst 7.

Approximately 47.7 grams of carrier B are placed in an impregnation vessel which is evacuated to about 28 inches-Hg absolute at ambient temperature. All of the impregnation mixture is placed promptly after zinc addition in the impregnation vessel under vacuum and allowed to contact the carrier for about one hour. The vessel is then opened and the impregnation mixture was drained from the carrier.

The first impregnated carrier is then roasted as described in Example 1.

The catalyst product is analyzed to contain 20.2 weight percent silver and 1013 parts per million by weight of potassium and 992 parts per million by weight of zinc.

The performance of this catalyst, catalyst 7, is determined using a 2-inch backmix autoclave as described above. Approximately 47.7 grams of catalyst 7 are charged to the autoclave. The feed composition and reaction conditions are as described in Example 6. The performance of catalyst 7 is summarized in Table 2.

## EXAMPLE 8

The preparation technique for the catalyst used in Example 8 is as follows. An impregnation solution is prepared by mixing 22.25 grams of ethylenediamine (high purity grade) with 22.05 of distilled water. Then 22.29 grams of oxalic acid dihydrate (reagent grade) are slowly added to the mixture at ambient conditions. The addition of the oxalic acid dihydrate is at a rate that the exotherm does not cause the temperature of the solution to rise above 40°C. Then 39.04 grams of silver oxide are added followed by 7.81 of monoethanolamine (Fe and Cl free). About 0.470 gram of $KNO_3$ in 10 ml water and 0.800 grams of $Ni(CHOO)_2.2H_2O$ are added. Distilled water is then added to adjust the volume to 125 milliliters.

Approximately 47.7 grams of carrier B are placed in an impregnation vessel which is evacuated to about 28 inches-Hg absolute at ambient temperature. All 125 ml of the impregnation is placed promptly after nickel addition in the impregnation vessel under vacuum and allowed to contact the carrier for about one hour. The vessel is then opened and the impregnation solution was drained from the carrier.

The first impregnated carrier is then roasted as described in Example 1.

The catalyst product is analyzed to contain 20.0 weight percent silver and 1000 parts per million by weight of potassium and 1458 parts per million by weight of nickel.

The performance of this catalyst, catalyst is determined using a 2-inch backmix autoclave as described above. Approximately 46.2 grams of catalyst 8 are charged to the autoclave. The feed composition and reaction conditions are as described in Example 6. The performance of catalyst 8 is summarized in Table 2.

## EXAMPLE 9

The preparation technique for the catalyst used in Example 9 is as follows. An impregnation solution is prepared by mixing 22.33 grams of ethylenediamine (high purity grade) with 22.13 of distilled water. Then 22.36 grams of oxalic acid dihydrate (reagent grade) are slowly added to the mixture at ambient conditions. The addition of the oxalic acid dihydrate is at a rate that the exotherm does not cause the temperature of the solution to rise above 40°C. Then 39.17 grams of silver oxide are added followed by 7.83 of monoethanolamine (Fe and Cl free). About 0.472 gram of $KNO_3$ in 10 ml water and 1.046 grams of $Cu(NO_3)_2$ in 10 ml water are added. Distilled water is then added to adjust the volume to 125 milliliters.

Approximately 47.7 grams of carrier B are placed in an impregnation vessel which is evacuated to about 28 inches-Hg absolute at ambient temperature. All 120 ml of the impregnation mixture promptly after copper addition is placed in the impregnation vessel under vacuum and allowed to contact the carrier for about one hour. The vessel is then opened and the impregnation mixture is drained from the carrier.

The first impregnated carrier is then roasted as described in Example 1.

The catalyst product is analyzed to contain 19.9 weight percent silver and 1000 parts per million by weight of potassium and 1215 parts per million by weight of copper.

The performance of this catalyst, catalyst 9, is determined using a 2-inch backmix autoclave as described above. Approximately 47.0 grams of catalyst 9 are charged to the autoclave. The feed composition and reaction conditions are as described in Example 6. The performance of catalyst 9 is

summarized in Table 2.

TABLE 2

| Catalyst | %EO | Efficiency, % | Temperature, °C | Day | ECl[1] | NO[2] |
|---|---|---|---|---|---|---|
| 1 | 1.80 | 85.3 | 255 | 20 | 4.4 | 8.0 |
| 1 | 1.52 | 85.3 | 255 | 30 | 4.5 | 8.3 |
| 1 | 1.35 | 85.1 | 255 | 40 | 4.9 | 8.2 |
| 2 | 1.74 | 85.4 | 255 | 30 | 4.7 | 8.3 |
| 3 | 1.95 | 85.0 | 255 | 40 | 6.2 | 10.6 |
| 4 | 1.98 | 85.8 | 255 | 20 | 6.0 | 8.0 |
| 5 | 2.05 | 85.4 | 255 | 20 | 4.0 | 5.0 |
| 6 | 0.44 | 79.0 | 270 | 20 | 20.0 | 15.0 |
| 7 | 0.82 | 83.5 | 270 | 20 | 20.0 | 15.0 |
| 8 | 0.74 | 80.2 | 270 | 20 | 20.0 | 15.0 |
| 9 | 0.72 | 84.8 | 270 | 20 | 20.0 | 15.0 |

(1) Concentrations of ethyl chloride in parts per million by volume.
(2) Concentrations of nitric oxide in parts per million by volume.

These results indicate that Catalyst 2 (containing iron), Catalyst 3 (containing iron), Catalyst 4 (containing nickel) and Catalyst 5 (containing rhenium) all produce increased amounts of ethylene oxide at comparable times on stream relative to Catalyst 1, which contains none of the present stability-enhancing components. This is an indication that all of Catalysts 2, 3, 4 and 5 have increased activity stability relative to Catalyst 1.

The efficiencies of Catalysts 2, 4 and 5 are higher at comparable times on stream relative to Catalyst 1. This is an indication that these catalysts are more efficiency stable than is Catalyst 1.

Comparing Examples 6 to 9 shows that Catalyst 7 (containing zinc), Catalyst 8 (containing nickel), and Catalyst 9 (containing copper) all produce greater amount of ethylene oxide and have higher efficiencies at comparable times on stream relative to Catalyst 6, which contains none of the present stability-enhancing components. This is an indication that all of Catalysts 7, 8 and 9 are more activity stable and more efficiency stable than is Catalyst 6.

## EXAMPLE 10

The preparation technique for the catalyst used in Example 10 is as follows. The first impregnation and roasting are as described in Example 2 except that 1300 milliliters of the impregnation solution is used to impregnate 600.1 grams of Carrier A.

An aqueous solution of the acid form of ethylenediaminetetraacetic acid ($H_4EDTA$) is prepared. This solution is heated to boiling and solid manganese (II) - carbonate is gradually introduced over a sufficient time to provide for complete dissolution. The amount of manganese (II)-carbonate is such that the mole ratio of Mn to $H_4EDTA$ is 1. The solution is then cooled and crystals of precipitate form. These crystals are recovered, and purified by washing with water and by being recrystallized from hot water. These crystals are determined to be the tetrahydrate of ethylene diamine tetraacetatomanganic II - acid, hereinafter $H_2MnEDTA$.

Another 128 milliliters of the impregnation solution are used for a second impregnation. About 1.06 grams of $KNO_3$ are added directly to this solution and 0.314 grams of $H_2MnEDTA$ are added.

Approximately 79.71 grams of the previously impregnated carrier is placed in an impregnation vessel which was evacuated to about 30 inches - Hg absolute at ambient temperature. All the 128 milliliters of the second impregnation solution are placed in the impregnation vessel under vacuum and allowed to contact the carrier for about 15 minutes. The vessel is then opened and the impregnation solution is drained from the carrier.

The second impregnated carrier is then roasted as described in Example 1. The catalyst product is analyzed to contain 33.2 weight percent silver, 1608 parts per million by weight of potassium and 217 parts per million by weight of manganese.

The performance of this catalyst, catalyst 10, is determined using a 2-inch backmix autoclave as described above. Approximately 61.1 grams of catalyst 10 are charged to the autoclave. The initial feed

composition and reaction conditions are as described in Example 1.

The performance of catalyst 10 is summarized as follows:

| EO, % | Efficiency, % | Temperature, °C | Day | ECl[(1)] | No[(2)] |
|---|---|---|---|---|---|
| 1.20 | 89.8 | 218.9 | 1 | 6.0 | 5.7 |
| 1.86 | 88.7 | 239.9 | 2 | 6.0 | 5.7 |

(1) Concentrations of ethyl chloride in parts per million by volume.
(2) Concentrations of nitric oxide in parts per million by volume.

These results indicate that catalysts prepared using complexing agents to complex metal-containing promoters such as manganese are very effective ethylene epoxidation catalysts. In addition, the use of such complexing agents allows a solution containing both silver and manganese to remain stable over a relatively long period of time, e.g., at least overnight.

While this invention has been described with respect to various specific examples and embodiments, it is to be understood that the invention is not limited thereto and that it can be variously practiced within the scope of the following claims.

## Claims

1. A catalyst for the manufacture of alkylene oxide by the epoxidation of alkene in the vapor phase in the presence of at least one efficiency-enhancing gaseous member of a redox-half reaction pair, said catalyst comprising impregnated silver on an inert, refractory solid support; an efficiency-enhancing amount of at least one efficiency-enhancing salt of a member of a redox-half reaction pair; and a sufficient amount of stability-enhancing component comprising at least one metal selected from the group consisting of iron, nickel, copper, ruthenium, rhodium, osmium, iridium, the rare earth metals, rhenium, antimony, gold, zinc, thallium, lead, tin and cadmium to enhance at least one of catalyst activity stability and catalyst efficiency stability as compared to a similar catalyst which does not contain the stability enhancing component, said comparison being under STANDARD ETHYLENE OXIDE PROCESS CONDITIONS.

2. The catalyst of claim 1 wherein said stability-enhancing component comprises at least one metal selected from the group consisting of iron, nickel, copper, ruthenium, rhodium, iridium, rhenium and zinc.

3. The catalyst of claim 1 wherein said stability-enhancing component comprises at least one metal selected from the group consisting of iron, nickel, copper, rhenium and zinc.

4. The catalyst of claim 1 in which the at least one efficiency-enhancing salt of a member of a redox-half reaction pair comprises alkali metal nitrate.

5. The catalyst of claim 4 in which the alkali metal nitrate comprises at least one of potassium nitrate and rubidium nitrate.

6. The catalyst of claim 5 in which the alkali metal nitrate comprises potassium nitrate.

7. The catalyst of claim 6 in which the efficiency-enhancing salt, calculated as cation, is about 0.01 to about 5 percent by weight based on the total weight of the catalyst.

8. The catalyst of claim 4 in which the efficiency-enhancing salt, calculated as cation, is about 0.01 to about 5 percent by weight based on the total weight of the catalyst.

9. A process for making alkylene oxide by the reaction of alkene and oxygen in which a gaseous stream containing alkene, oxygen, gas phase inhibitor and at least one efficiency-enhancing gaseous member of a redox-half reaction pair is fed under alkylene oxide producing conditions to a bed of catalyst of claim 1 and alkylene oxide is provided in effluent from the bed of catalyst.

**10.** The process of claim 9 wherein said stability-enhancing component of said catalyst comprises at least one metal selected from the group consisting of iron, nickel, copper, ruthenium, rhodium, iridium, rhenium and zinc.

**11.** The process of claim 9 wherein said stability-enhancing component of said catalyst comprises at least one metal selected from the group consisting of iron, nickel, copper, rhenium and zinc.

**12.** The process of claim 9 wherein said efficiency enhancing gaseous and salt members of a redox-half reaction pair comprise members of the same redox-half reaction.

**13.** The process of claim 9 wherein said at least one gaseous member of a redox-half reaction is NO, $NO_2$, $N_2O_3$,$N_2O_4$, $N_2O_5$, or a gas capable of forming one of the aforementioned gases under epoxidation conditions.

**14.** The process of claim 13 wherein said gas capable of forming said one of the aforementioned gases is a gas which forms NO and/or $NO_2$ under epoxidation conditions.

**15.** The process of claim 9 wherein said at least one efficiency-enhancing salt of a member of a redox-half reaction pair comprises potassium nitrate.

**16.** The process of claim 9 wherein said at least one gaseous member of a redox-half reaction pair comprises NO.

**17.** The process of claim 9 wherein said at least one gaseous member comprises NO and said at least one salt comprises potassium nitrate.

**18.** The process of claim 9 wherein said alkene comprises ethylene.

**19.** The process of claim 9 wherein said alkene comprises propylene.

**20.** A process for preparing a promoted supported silver catalyst for the production of ethylene oxide by the vapor phase oxidation of ethylene with an oxygen-containing gas comprising:

(a) impregnating a catalyst support with a solution comprising a liquid solvent, at least one of a silver salt and a silver complex and at least one metal-containing promoter at least a portion of which is present as a metal complex, said solution having enhanced solubility stability relative to the solubility stability of a similar solution in which none of the metal-containing promoter is complexed; and

(b) treating the impregnated support produced in step (a) to effect deposition of silver and said metal-containing promoter on said support.

**21.** The process of claim 20 wherein said metal-containing promoter includes at least one metal selected from the group consisting of the metals of Groups 1b, 2b, 3b, 4b, 5b, 6b, 7b and 8 of the Periodic Table, the rare earth metals, tin, antimony, lead, thallium and bismuth.

**22.** The process of claim 20 wherein said metal-containing promoter includes at least one metal selected from the group consisting of manganese, iron, cobalt, nickel, copper, gold, ruthenium, rhodium, osmium, iridium, zinc, cadmium, the rare earth metals, antimony, gold, zinc, thallium, lead, tin and cadmium.

**23.** The process of claim 20 wherein said metal-containing promoter includes manganese or cobalt.

**24.** The process of claim 20 wherein said metal-containing promoter includes manganese.

**25.** The process of claim 20 wherein at least one complexing agent effective to form said metal complex is present in said solution in an amount of at least about 50% of that amount needed so that all of said metal-containing promoter in said solution is present as said metal complex.

**26.** The process of claim 20 wherein at least one complexing agent effective to form said metal complex is present in said solution in an amount in the range of about 50% to about 400% of that amount needed

so that all of said metal-containing promoter in said solution is present as said metal complex.

**27.** The process of claim 20 wherein said metal complex is derived from a complexing agent selected from the group consisting of ethylenediaminetetraacetic acid; N, N'-ethylenediaminediacetic acid; N-hydroxyethylethylenediaminetriacetic acid; diethylenetriaminepentaacetic acid; nitrilotriacetic acid; N-hydroxyethyliminodiacetic acid; N-dihydroxyethylglycine and mixtures thereof.

**28.** A process for preparing a promoted supported silver catalyst for the production of ethylene oxide by the vapor phase oxidation of ethylene with an oxygen-containing gas comprising:

(a) impregnating a catalyst support with a solution comprising a liquid solvent, at least one of a silver salt and a silver complex, at least one metal-containing promoter, and at least one complexing agent effective to complex the metal species of said metal-containing promoter in an amount sufficient to enhance the solubility stability of said metal-containing promoter in said solution relative to the solubility stability of said metal-containing promoter in a similar solution in which the metal-containing promoter is not complexed with said complexing agent and which does not include said complexing agent; and

(b) treating the impregnated support produced in step (a) to effect deposition of said metal-containing promoter on said silver-containing support.

**29.** The process of claim 28 wherein said metal-containing promoter includes at least one metal selected from the group consisting of the metals of Groups 1b, 2b, 3b, 4b, 5b, 6b, 7b and 8 of the Periodic Table, the rare earth metals, tin, antimony, lead, thallium and bismuth.

**30.** The process of claim 28 wherein said metal-containing promoter includes at least one metal selected from the group consisting of manganese, iron, cobalt, nickel, copper, gold, ruthenium, rhodium, osmium, iridium, zinc, cadmium, the rare earth metals, antimony, gold, zinc, thallium, lead, tin and cadmium.

**31.** The process of claim 28 wherein said metal-containing promoter includes manganese and cobalt.

**32.** The process of claim 28 wherein said metal-containing promoter includes manganese.

**33.** The process of claim 28 wherein said complexing agent is present in said solution in an amount of at least about 50% of that amount needed so that all of said metal-containing promoter in said solution is present as a metal complex.

**34.** The process of claim 28 wherein said complexing agent is present in said solution in an amount in the range of about 50% to about 400% of that amount needed so that all of said metal-containing promoter in said solution is present as a metal complex.

**35.** A process for making alkylene oxide by the reaction of alkene and oxygen comprising contacting a gaseous stream containing alkene, oxygen, gas phase inhibitor and at least one efficiency-enhancing gaseous member of a redox-half reaction pair under alkylene oxide producing conditions in the presence of a bed of catalyst containing silver metal on an inert, refractory solid support and an efficiency-enhancing amount of at least one efficiency-enhancing salt of a member of a redox-half reaction pair, and providing an effluent from the bed of catalyst containing alkylene oxide, wherein the bed of catalyst contains sufficient Stability Catalyst to enhance the rate of start-up of the bed of catalyst to produce alkylene oxide relative to a similar bed of catalyst which contains no Stability Catalyst, said Stability Catalyst comprising at least one metal selected from the group consisting of iron, nickel, copper, ruthenium, rhodium, osmium, iridium, the rare earth metals, rhenium, antimony, gold, zinc, thallium, lead, tin, cadmium, manganese and cobalt.

**36.** A process for making alkylene oxide by the reaction of alkene and oxygen comprising contacting a gaseous stream containing alkene, oxygen, gas phase inhibitor and at least one efficiency-enhancing gaseous member of a redox-half reaction pair under alkylene oxide producing conditions in the presence of a fixed bed of catalyst containing silver metal on an inert, refractory solid support and an efficiency-enhancing amount of at least one efficiency-enhancing salt of a member of a redox-half reaction pair, and providing an effluent from the fixed bed of catalyst containing alkylene oxide, wherein

the fixed bed of catalyst contains sufficient Stability Catalyst to increase catalyst effectiveness along the length of the fixed bed of catalyst relative to a similar fixed bed of catalyst which contains no Stability Catalyst, said Stability Catalyst comprising at least one metal selected from the group consisting of iron, nickel, copper, ruthenium, rhodium, osmium, iridium, the rare earth metals, rhenium, antimony, gold, zinc, thallium, lead, tin, cadmium, manganese and cobalt.

**37.** A process for making alkylene oxide by the reaction of alkene and oxygen comprising contacting a gaseous stream containing alkene, oxygen, gas phase inhibitor and at least one efficiency-enhancing gaseous member of a redox-half reaction pair under alkylene oxide producing conditions in the presence of a bed of catalyst containing silver metal on an inert, refractory solid support and an efficiency-enhancing amount of at least one efficiency-enhancing salt of a member of a redox-half reaction pair, and providing an effluent from the bed of catalyst containing alkylene oxide, wherein at least one component of said gaseous stream or a portion or all of said gaseous stream is contacted with a Stability Catalyst conditions effective to provide an enhanced alkylene epoxidation process relative to using a similar gaseous stream none of which is contacted with a Stability Catalyst, said Stability Catalyst comprising at least one metal selected from the group consisting of iron, nickel, copper, ruthenium, rhodium, osmium, iridium, the rare earth metals, rhenium, antimony, gold, zinc, thallium, lead, tin, cadmium, manganese and cobalt.

European Patent Office

# EUROPEAN SEARCH REPORT

Application Number

**EP 91 20 2652**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 266 852 (SHELL)<br>* column 1, line 38 - line 51 *<br>* column 3, line 11 - line 49 *<br>* column 4, line 16 - line 53 *<br>* column 5, line 8 - line 13 *<br>* claims; example * * | 1-8 | B 01 J 23/66<br>B 01 J 23/68<br>C 07 D 301/10 |
| Y | (* column 6, line 1 - column 7, line 15 *) | 9-37 | |
| Y | GB-A-2 161 480 (MITSUI TOATSU CHEM INC)<br>* page 3, line 10 - line 60 *<br>* claims; examples 1-5; tables 1,2 * * | 9-19 | |
| D,Y | US-A-4 455 392 (WARNER ET AL)<br>* column 4 - column 8 *<br>* example 1 * * | 20-37 | |
| A | GB-A-2 042 361 (TEXACO DEV) | | |
| D,A | GB-A-2 014 133 (ICI) | | |

TECHNICAL FIELDS SEARCHED (Int. Cl.5)

B 01 J
C 07 D

The present search report has been drawn up for all claims

| Place of search | Date of completion of search | Examiner |
|---|---|---|
| The Hague | 22 January 92 | LO CONTE C. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document